# EUROPEAN PATENT SPECIFICATION

(11) **EP 1 652 508 B1**
(45) Date of publication and mention of the grant of the patent: **04.12.2013**
(21) Application number: 04771068.6
(22) Date of filing: 30.07.2004
(51) Int. Cl.: A61K 9/70, A61K 31/138

(54) **ADHESIVE PATCH**
KLEBEPFLASTER
PATCH ADHESIF

(30) Priority: 31.07.2003 JP 2003283301
(43) Date of publication of application: 03.05.2006
(73) Proprietor: Hisamitsu Pharmaceutical Co., Inc., Tosu-shi Saga 841-0017 (JP)
(72) Inventor: TATEISHI, Tetsuro Hisamitsu Pharmaceutical Co Inc., Tsukuba-shi Ibaraki 3050856 (JP); AMANO, Satoshi Hisamitsu Pharmaceutical Co. Inc., Tsukuba-shi, Ibaraki 3050856 (JP); HONMA, Sachiko Hisamitsu Pharmaceutical Co., Inc., Tsukuba-shi, Ibaraki 3050856 (JP); HIGO, Naruhito Hisamitsu Pharmaceutical Co. Inc., Tsukuba-shi, Ibaraki 3050856 (JP)
(74) Representative: Thomson, Craig Richard
(86) International application number: PCT/JP2004/010908
(87) International publication number: WO 2005/011662

(56) References cited:
- WO-A1-01/07018
- WO-A1-02/069942
- WO-A2-02/03969
- JP-A- 3 261 722
- JP-A- 9 301 854
- JP-A- 10 179 711
- JP-A- 11 152 224
- JP-A- 2003 313 122
- JP-A- 2004 010 525

## Description

### [Technical Field]

The invention relates to an adhesive patch comprising bisoprolol which is a β-blocker.

### [Background Art]

As administration methods for drugs, a peroral administration using tablets, capsules, syrups and the like has been made in many drugs. However, in case of the peroral administration, there were drawbacks that it lacked in duration of effect, an unnecessary high blood concentration was recognized for a while after the administration, whereby a side effect occurred easily, and so on. In order to eliminate such drawbacks of the peroral administration, the development for percutaneous absorption type pharmaceutical preparations has actively been carried out. The percutaneous absorption type pharmaceutical preparations cover not only those drawbacks, but are expected to have merits such as reduction of administration frequency, improvement of compliance and simplicity of administration as well as its discontinuation, and are known to be useful particularly in aged and infantile patients.

Bisoprolol is highly β₁-selective antagonisy and a β-blocker which does not have an Intrinsic sympathomimetic activity and a membrane stabilizing activity. At present, in clinical treatment it is used only in a peroral preparation as a therapeutic drug for hypertension, angina pectoris, ventricular premature beat, etc.
Although bisoprolol is relatively little in the effect to bronchus due to the high ß₁ selectivity, there are cases that symptoms such as bradycardia, dizziness and physical weariness occur, therefore, development of a percutaneous pharmaceutical preparation has been desired from the viewpoint of stabilization of a blood concentration and sustainability of the effect.
Although an invention concerning an apparatus to administer a stereospecific drug using an electrotransport way is disclosed as one of techniques to administer drugs percutaneously (for example, see US 6136327), a skin irritation occurs due to use of an electric energy, and further, there is a drawback that it lacks in easiness of administration.
Therefore, there was need for a percutaneous absorption type pharmaceutical preparation containing bisoprolol which could be administered easily, in which the stability of bisoprolol was secured with easiness and mild skin irritation as well as its blood concentration could stably be kept.
Further, although an adhesive patch comprising bisoprolol, in which an acrylic adhesive polymer is used in a pressure-sensitive adhesive layer, is disclosed after application that becomes a base of the right of priority in the application concerned (see JP, A1, 2003-313122), the acrylic adhesive polymer is a copolymer that consists of an alkyl (meth) acrylate and a copolymerizable monomer containing neither of a carboxyl group nor a sulfo group; it has been clear from the research by the inventors that the stability of bisoprolol could not maintained by a copolymer that consists of an alkyl (meth) acrylate and a copolymerizable monomer not containing the carboxyl group.
In addition, WO 02/03969 describes a transdermal therapeutic system comprising a surface layer which is impervious with respect to an active ingredient; a self-adherent matrix layer or a plurality of matrix layers, wherein the exposed matrix layer is, at least self-adherent when the system is applied. Said system also comprises a pull-off protective coating, whereby the matrix layer contains one or more active ingredients and/or one or more biologically active substances and highly dispersed silicon dioxide. Said system contains silicone dioxide in order to increase skin permeation.

### [Disclosure of the Invention]

### [Problem to Be Solved by the Invention]

In an adhesive patch comprising bisoprolol and/or a pharmaceutically acceptable salt thereof, the problem of the invention is to provide an adhesive patch which is excellent in a skin adhesiveness and can keep a blood concentration constant by making a specific pressure-sensitive adhesive layer having a self-adhesive force.

### [Means for Solving Problem]

During extensive research to solve the above problems, the inventors succeeded in finding a specific composition of pressure-sensitive adhesive layer, which can dissolve an effective amount of bisoprolol and stably contain it for a long time with keeping adherence, and accomplished the invention as a result of further investigation.
In addition, the inventors found that it was possible to enhance penetration through the skin of bisoprolol by letting a specific percutaneous absorption promoter be contained in the specific composition of pressure-sensitive adhesive layer.

The present invention relates to an adhesive patch having a pressure-sensitive adhesive layer comprising bisoprolol and/or a pharmaceutically acceptable salt thereof, wherein the composition of pressure-sensitive adhesive layer contains an acrylic polymer obtained by copolymerizing a (meth) acrylic ester with a (meth)acrylic acid having a carboxyl group and an elastomeric polymer, as claimed hereafter.
Preferred embodiments of the invention are set forth in the dependent claims.
In addition, the invention relates to the above adhesive patch, wherein the acrylic polymer contains substantially no alcoholic hydroxyl group in molecules.
Furthermore, the invention relates to the above adhesive patch, wherein the (meth) acrylic ester constituting the acrylic polymer is 2-ethylhexyl acrylate.

The invention relates to the above adhesive patch, wherein the acrylic polymer is a 2-ethylhexyl acrylate·vinyl acetate acrylic acid copolymer.
In addition, the invention relates to the above adhesive patch, wherein the elastmeric polymer is a hydrophobic polymer.

Preferably, the elastmeric polymer is one kind selected from a group consisting of styrene-isoprene-styrene block copolymer, polyisobutylene, isoprene rubber, styrene-butadine-styrene block copolymer, styrene-butadine rubber and silicone rubber, more preferably styrene-isoprene-styrene block copolymer.
In addition, the invention relates to the above adhesive patch, wherein preferably the percutaneous absorption promoter is one or more kinds selected from a group consisting of lauryl alcohol, myristyl alcohol, oleyl alcohol, isostearyl alcohol, diethyl sebacate, lauric diethanolamide, isopropylmyristate, glycerol monocaprate, glycerol monolaurate, glycerol monooleate, sorbitan monolaurate, propylene glycol monolaurate, polyoxyethylene lauryl ether, pyrothiodecane, an organic acid and a pharmaceutically acceptable salt thereof.
Furthermore, the invention relates to the above adhesive patch, wherein the organic acid and/or the pharmaceutically acceptable salt thereof are at least one kind selected from a group consisting of acetic acid, propionic acid, lactic acid, salicylic acid and pharmaceutically acceptable salts thereof.

The invention relates to the above adhesive patch, wherein the percutaneous absorption promoter is isopropyl myristate.
The invention relates to the above adhesive patch, wherein it contains further sodium acetate as the percutaneous absorption promoter.
Further, the invention relates to the above adhesive patch, wherein the drug is bisoprolol hemifumarate.
Furthermore, the invention relates to the above adhesive patch, wherein the penetration rate through the skin of bisoprolol and/or a pharmaceutically acceptable salt thereof is 3-300 µg/h·cm².

By having the above constitution, the invention provides an adhesive patch which can stably keep bisoprolol in a preparation, and further, is excellent in penetration through the skin, while the blood concentration does not transiently rise, therefore, the bisoprolol concentration can be kept constant without reaching a concentration which manifests symptoms such as bradycardia.

### [Best Embodiment for Carrying out the Invention]

In the following, the adhesive patch of the invention is illustrated in more detail.
The adhesive patch of the invention indicates an adhesive patch containing at least a backing and a pressure-sensitive adhesive layer composition and compromises the so called external patch of reservoir type and external patch of matrix type. Comparing an external patch of reservoir type and an external patch of matrix type, generally, the external patch of matrix type, in which a composition of pressure-sensitive adhesive layer having a self adhesive force adheres directly through the skin, is more excellent in adhesiveness and is also excellent in penetration of a drug to the skin, therefore, in the following an adhesive patch of the invention is mainly explained taking an adhesive patch of matrix type as an example, though it is not limited this.

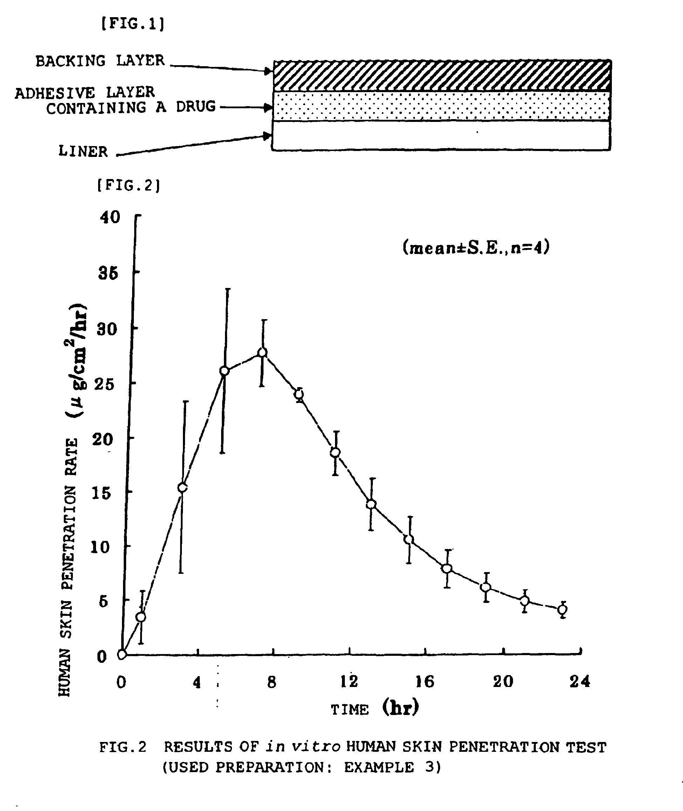

The adhesive patch of the invention is one which contains a composition of pressure-sensitive adhesive layer and bisoprolol and/or a pharmaceutically acceptable salt thereof as a drug, wherein said drug is released at a pharmacologically effective rate, while the penetration rate through the skin per hour is 3-300 µg/cm².
Typically, it is in a form which consists of a hydrophobic matrix (pressure-sensitive adhesive layer) containing a drug (bisoprolol and/or a pharmaceutically acceptable salt thereof) as is shown in Fig.1, and a backing on its back. Although this pressure-sensitive adhesive layer preferably has an adhesive force to maintain an effective area on a surface of the skin for at least 12 or more hours without problem for therapy, in a case that it is difficult, it is possible to use a sheet type cover which has a larger area compared with a layer containing the drug and also has an adhesive force.

Bisoprolol which can be used in the invention may be a free formoritssalt. In case of using the salt, it is not particularly limited if it is a pharmaceutically acceptable salt, however, preferably fumarate, hydrochloride, sulfonate, mesylate, citrate,tartarate,maleate oracetate. Amongthese, bisoprolol hemifumarate is more preferable.

Generally, when using bisoprolol hemifumarate as a peroral preparation, 5-10 mg is administered as a usual dose in one time a day. That is, use of such a dose as a peroral preparation is a necessary dose for obtaining a pharmaceutical effect of bisoprolol.
Meanwhile, even if a blood concentration of a drug in case of using it in a peroral preparation becomes clear, in case of using it in a percutaneous absorption it is not possible to easily infer whether a blood concentration to show almost same pharmaceutical effect compared with a case of a peroral administration is obtained at any penetration rate through the skin, because penetration through the skin shows totally different behavior depending on each drug.

In such circumstances, it was found that by pharmacokinetical consideration of bisoprolol the adhesive patch of the invention needed a penetration rate through the skin at 3-300 µg/cm² per hour in order to maintain a blood concentration effective for therapy by bisoprolol, and further, an adhesive patch having a composition of pressure-sensitive adhesive layer to attain the target figure was made.

Here, although the penetration rate through the skin at 3-300 µg/cm² per hour depends on an application area of an adhesive patch, the invention was accomplished considering that a general area of the adhesive patch is 1-100cm² (in case of less than 1cm², treatment becomes difficult when pasting, and in case of exceeding 100cm² an uncomfortable feeling becomes a problem when pasting). Further, the penetration rate through the skin mentioned here means the maximum penetration rate through the skin during an application of the adhesive patch to a human skin.

From a viewpoint that a composition of pressure-sensitive adhesive layer of the patch of the invention, which contains bisoprolol, has a sufficient drug solubility to obtain the above penetration rate through the skin and can maintain the stability of drug bisoprolol in the composition of pressure-sensitive adhesive layer, an acrylic polymer obtained by coplymerizing a (meth) acrylic ester with a (meth) acrylic acid having a carboxyl group is used.

As a (meth) acrylic ester constituting the acrylic polymer, illustrative are 2-ethylhexyl acrylate, methyl acrylate, butyl acrylate, hydroxyethyl acrylate, 2-ethylhexyl (meth) acrylate, and the like, though 2-ethylhexyl acrylate is particularly preferable.
In addition, as a (meth) acrylic acid having a carboxyl group constituting the acrylic polymer, illustrative are acrylic acid, methacrylic acid and the like, though acrylic acid is preferable. When using a copolymer containing a acrylic acid polymer having a carboxyl group in molecules, it is possible to maintain the stabilityof bisoprolol of an active substance andalso to improve an adhesive force, or in case of carrying out cross-linking as required, it can be used as a reaction site.

For a further stability of the active substance, an acrylic polymer used as a base of the pressure-sensitive adhesive layer is preferably a copolymer which contains substantially no alcoholic hydroxyl group in molecules. By use of the acrylic polymer which contains substantiallyno alcoholic hydroxyl group in molecules, the preparation stability of bisoprolol (or its salt) can be improved, while the reason is not sure, however, in case of existence of the alcoholic hydroxyl group it is anticipated that it is due to appearance of some interaction between the alcoholic hydroxyl group and bisoprolol.

As such acrylic polymer, it is not particularly limited if it has substantially no alcoholic hydroxyl group and has carboxyl group, illustrative are, for example, 2-ethylhexyl acrylate·vinyl acetate·acrylic acid copolymer, acrylic acid·octyl acrylate copolymer, Duro-Tak87-2852, Duro-Tak87-2194, Duro-Tak87-2196, Duro-Tak87-2353, Duro-Tak87-2051, Duro-Tak87-2052, Duro-Tak87-2054, Duro-Tak87-2825, Duro-Tak87-2677, Duro-Tak80-1196 (manufactured by National Starch and Chemicals Co., Ltd.) and the like.
Further, in a preparation step of the above acrylic polymer, when a monomer having hydroxyl group in the monomer of a starting material exists in a minute amount as impurities, or a side reaction such as thermal degradation occurs in case of polymerization, there is a case that the hydroxyl group resulting from the impurities is introduced in an obtained acrylic polymer, however, such a acrylic polymer is comprised in the acrylic polymer having substantially no alcoholic hydroxyl group in molecules and having carboxyl group so far as it does not impair features which an adhesive patch of the invention has.

In addition, the pressure-sensitive adhesive layer related to the invention contains an elastmeric polymer except the above acrylic polymer. By containing the elastmeric polymer in the pressure-sensitive adhesive layer, adhesiveness of a preparation can be controlled. The elastmeric polymer used in the invention contains both of a natural or synthetic elastmeric polymers. Preferable examples of such elastmeric polymer include styrene-isoprene-styrene block copolymer, isoprene rubber, polyisobutylene, styrene-butadine-styrene block copolymer, styrene-butadine rubber, polysiloxane and the like. Among these, styrene-isoprene-styrene block copolymer and/or polyisobutylene are preferably used.

Although the hydrophobic polymer may be used in one kind or by mix of two or more kinds, mix of the acrylic polymer and the elastmeric polymer is further preferable because a preparation satisfying both of a drug penetration through the skin and physical properties of the preparation is given. Considering formation of a pressure-sensitive adhesive layer and sufficient penetration, the blend amount of these polymers based on the weight of the total composition may be 5-90 wt.%, preferably 10-70 wt.%, more preferably 10-50 wt.%.

In the invention it is desired to let an organic acid be contained in the pressure-sensitive adhesive layer in a case that a form of an active substance is a pharmaceutically acceptable acid-addition salt, and as organic acids used, illustrative are aliphatic (mono-, di-, tri-)carboxylic acids (e.g., acetic acid, propionic acid, isobutylic acid, caproic acid, caprylic acid, lactic acid, maleic acid, pyruvic acid, oxalic acid, succinic acid, tartaric acid and the like), aromatic carboxylic acids (e.g., phthalic acid, salicylic acid, bezoic acid, acetyl salicylic acid and the like), alkyl sulfonic acids (e.g., methanesulfonic acid, ethanesulfonic acid, propyl sulfonic acid, butanesulfonic acid,polyoxyethylenealkylether sulfonic acid), alkyl sulfonic acid derivatives (e.g., N-2-hydroxyethyl-piperidine-N'-2-ethane sulfonic acid (hereinafter abbreviated as HEPES) and cholic acid (e.g., dehydrocholic acid). Among them acetic acid, propionic acid, lactic acid and salicylic acid are preferable, and acetic acid is in particular preferable. In addition, in these organic acids, salts thereof or a mixture with a salt may be used. By letting such an organic acid be contained in the pressure-sensitive adhesive layer composition, it becomes possible to increase penetration through the skin.

When using particularly fumaric acid salt of bisoprolol, a sufficient penetration rate through the skin shown above can be obtained by letting acetic acid salt be contained in the composition of pressure-sensitive adhesive layer.

Considering a sufficient penetration amount as the adhesive patch and the irritating properties to the skin, these organic acid can be blended preferably in the amount of 0.01-20 wt.% based on the weight of the total composition of pressure-sensitive adhesive layer, more preferably 0.1-15wt.%, in particular preferably 0.1-10 wt.%.

An absorption promoter may be contained in the pressure-sensitive adhesive layer of the adhesive patch of the invention, and as an absorption promoter, any compound in which an absorption promoting effect is shown may be used. Examples include C₆-C₂₀ fatty acids, fatty alcohols, fatty acid esters, amides or ethers, aromatic organic acids, aromatic alcohols, aromatic organic acid esters or ethers (these may be saturated or unsaturated, and may be cyclic, straight or branched), furthermore lactic acid esters, acetic acid esters, monoterpene compounds, sesquiterpene compounds, Azone, Azone derivatives, pirotiodecane, glycerol fatty acid esters, propylene glycol fatty acid esters, sorbitan fatty acid esters (Span type), polysorbates (Tween type), polyethylene glycol fatty acid esters, polyoxyethylene hydrogenated castor oils (HCO type), polyoxyethylene alkyl ethers, sucrose fatty acid esters, plant oils.

Specifically, caprylic acid, capric acid, caproic acid, lauric acid, myristic acid, palmitic acid, stearic acid, isostearic acid, oleic acid, linoleic acid, linolenic acid, lauryl alcohol, myristyl alcohol, oleyl alcohol, isostearyl alcohol, cetyl alcohol, methy laurate, hexyl laurate, diethyl sebacate, lauric diethanolamide, isopropylmyristate, myristyl myristate, octyldodecyl myristate, cetyl palmitate, salicylic acid, methyl salicylate, ethylene glycol salicylate, cinnamic acid, methyl cinnamate, cresol, cetyl lactate, lauryl lactate, ethyl acetate, propyl acetate, geraniol, thymol, eugenol, terpineol, 1-menthol, borneol, d-limonene, isoeugenol, isoborneol, nerol, dl-camphor, glycerol monocaprylate, glycerol monocaprate, glycerol monolaurate, glycerol monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, HCO-60, pyrothiodecane and olive oil are preferable, and lauryl alcohol, myristyl alcohol, oleyl alcohol, isostearyl alcohol, diethyl sebacate, lauric diethanolamide, isopropylmyristate, glycerol monocaprate, glycerol monolaurate, glycerol monooleate, sorbitan monolaurate, propylene glycol monolaurate, polyoxyethylene lauryl ether and pyrothiodecane are particularly preferable, and isopropyl myristate is more preferable.

The absorption promoter may be used by mix of two or more kinds, and considering a sufficient penetration as the adhesive patch and irritating properties such as erythema, edema and the like, it can be blended preferably in 0.01-40 wt.% based on the weight of the total composition of pressure-sensitive adhesive layer, more preferably 0.05-30 wt.%, in particular preferably 0.1-20 wt.%.

A plasticizer may be contained in the pressure-sensitive adhesive layer of the patch of the invention. As usable plasticizers, illustrative are petroleum oils (e.g., paraffin type process oil, naphthalene type process oil, aromatic type process oil), squalane, squalene, vegetable oils (e.g., olive oil, camellia oil, castor oil, tall oil, peanut oil), siliconeoil, dibasicacidesters (e.g., dibutylphthalate, dioctyl phthalate), liquefied rubber (e.g., polybutene, liquefied isoprene rubber), liquefied fatty acid esters (isopropyl myristate, hexyl laurate, diethyl sebacate, diisopropyl sebacate), diethylene glycol, polyethylene glycol, glycol salicylate, propylene glycol, dipropylene glycol, triacetin, triethyl citrate, crotamiton. In particular, liquid paraffin, liquefied polybutene, isopropyl myristate, diethyl sebacate and hexyl laurate are preferable.

These components may be used by mix of two or more kinds, and considering a sufficient penetration and maintenance of a sufficient cohesive force as the adhesive patch, the blend amount of such plasticizers based on the total composition in the pressure-sensitive adhesive layer can be 1-70 wt.% in total, preferably 3-50 wt.%, more preferably 5-40 wt.%.

A tackifying resin may desirably be contained in the pressure-sensitive adhesive layer of the invention in case of lacking in an adhesive force possible for application for at least 12 hours , and as usable tackifying resins, illustrative are rosin derivatives (e.g., rosin, glycerol esters of rosin, hydrogenated rosin, glycerol esters of hydrogenated rosin, pentaerythritol esters of rosin), alicyclic saturatedhydrocarbonresins (e.g., Arcon P 100, Arakawa Chemical Industries, Ltd.), aliphatic hydrocarbon resins (e.g., Quintone B170, Zeon Coorporation), terpene resins (e.g., Clearon P-125, Yasuhara Chemical), maleic acid resins. In particular, glycerol esters of hydrogenated rosin, alicyclic saturated hydrocarbon resins and terpene resins are preferable.

Considering a sufficient adhesive force as the adhesive patch and irritating properties to the skin at the time of dissection, the blend amount of such tackifying resins based on the total composition in the pressure-sensitive adhesive layer can be 1-70 wt.%, preferably 5-60 wt.%, more preferably 10-50 wt.%.

In addition, as required, antioxidants, fillers, cross-linking agents, preservatives or UV absorbers can be used. As antioxidants, tocopherol and its ester derivatives, ascorbic acid, ascorbic acid-stearic acid ester, nordihydroguaretic acid, dibutyl hydroxy toluene (BHT), butyl hyroxy anisole are desirable. As fillers, calcium carbonate, magnesium carbonate, silicate (e.g., aluminum silicate, magnesium silicate), silicic acid, barium sulfate, calcium sulfate, calcium zincate, zinc oxide, titanic oxide are desirable. As cross-linking agents, thermosetting resins such as amino resins, phenol resins, epoxy resins, alkyd resins and unsaturated polyesters, isocyanate compounds, block isocyanate compounds, organic type cross-linking agents, and inorganic type cross-linking agents such as metals or metal compounds, are desirable. As preservatives, ethyl p-hydroxy benzoate, propyl p- hydroxy benzoate, butyl p- hydroxy benzoate and the like are desirable. As UV absorbers, p-amino benzoic acid derivatives, anthranilic acid derivatives, salicylic acid derivatives, coumarin derivatives, amino acid type compounds, imidazoline derivatives, pyrimidine derivatives, dioxane derivatives are desirable.

Such antioxidants, fillers, cross-linking agents, preservatives and UV absorbers can be blended preferably with an amount of not more than 10 wt.% in total based on the weight of the total composition in the pressure-sensitive adhesive layer of the adhesive patch, more preferably not more than 5 wt.% and in particular preferably not more than 2 wt.%.

The drug-containing pressure-sensitive adhesive layer containing the composition described above can be prepared by any method. For example, a base composition containing a drug is heat-melted, coated on a removable paper or a backing, followed by affixing to the backing or the removable paper to give the present preparations. Also, base components containing a drug are dissolved in solvent such as toluene, hexane or ethyl acetate, spreaded on the removable paper or the backing, dried to remove solvent, followed by affixing to the backing or the removable paper to give the present preparations.

Although the adhesive patch of the invention is typically an adhesive patch shown in Fig. 1, as to the backing, an elastic or a non-elastic backing can be used. For example, it can be selected from woven fabric, knitted fabric, nonwoven fabric, polyurethane, polyester, polyvinyl acetate, polyvinylidene chloride, polyethylene, polyethylene terephthalate, aluminum sheet, or composite materials thereof.
In addition, although the liner is not particularly limited if it can protect a pressure-sensitive adhesive layer till applying an adhesive patch to the skin, specifically, films such as polyesters (polyethylene terephthalate, etc.), polyvinyl chloride and polyvinylidene chloride, a laminated film of a high-quality paper with polyolefin, may be used. In these liners, if a silicone treatment is applied to the surface of the side attached to the pressure-sensitive adhesive layer, it is preferable because operation in case of releasing the liner from the preparation is facilitated.

### [Example]

In the following, the invention is explained in more detail by the examples. Further, in the examples, all % mean % by weight.

### (Example 1)

| | |
|---|---|
| 2-Ethylhexyl acrylate·vinyl acetate·acrylic acid copolymer | 19.3% |
| Styrene-isoprene-styrene block copolymer | 8.4% |
| Alicyclic saturated hydrocarbon resin | 40.8% |
| Liquid paraffin | 10.5% |
| Isopropyl myristate | 10.5% |
| Bisoprololhemifumarate | 10.5% |
| | Total amount 100.0% |

Bisoprolol hemifumarate, isopropyl myristate and liquid paraffin were put in a mortar and mixed thoroughly. The mixture was mixed with a solution in which 2-ethylhexyl acrylate·vinyl acetate·acrylic acid copolymer, styrene-isoprene-styrene block copolymer and alicyclic saturated hydrocarbon resin (Arcon P 100, manufactured by Arakawa Chemical Industries, Ltd.) were dissolved in toluene and ethyl acetate. After the coating solution obtained was coated on a removable film made by polyethylene terephthalate, solvent was removed by drying, followed by affixing to a backing made by polyethylene terephthalate to give the adhesive patch of the invention.

### (Example 2)

| | |
|---|---|
| 2-Ethylhexyl acrylate·vinyl acetate·acrylic acid copolymer | 20.6% |
| Styrene-isoprene-styrene block copolymer | 8.9% |
| Alicyclic saturated hydrocarbon resin | 43.3% |
| Liquid paraffin | 11.1% |
| Sodium acetate | 5.0% |
| Bisoprololhemifumarate | 11.1% |
| | Total amount 100.0% |

Except adding sodium acetate instead of isopropyl myristate as a percutaneous absorption promoter, the adhesive patch of the invention was prepared in the same way as the example 1.

### (Example 3)

| | |
|---|---|
| 2-Ethylhexyl acrylate·vinyl acetate·acrylic acid copolymer | 18.5% |
| Styrene-isoprene-styrene block copolymer | 8.0% |
| Alicyclic saturated hydrocarbon resin | 39.0% |
| Liquid paraffin | 10.0% |
| Isopropyl myristate | 10.0% |
| Sodium acetate | 4.5% |
| Bisoprololhemifumarate | 10.0% |
| | Total amount 100.0% |

According to the above formula in which isopropyl myristate and sodium acetate were added as percutaneous absorption promoters, the adhesive patch of the invention was prepared in the same way as the example 1.

### (Comparative Example 4)

| | |
|---|---|
| 2-Ethylhexyl acrylate·vinyl acetate·hydroxyethyl acrylate copolymer | 18.5% |
| Styrene-isoprene-styrene block copolymer | 8.0% |
| Alicyclic saturated hydrocarbon resin | 39.0% |
| Liquid paraffin | 10.0% |
| Isopropyl myristate | 10.0% |
| Sodium acetate | 4.5% |
| Bisoprololhemifumarate | 10.0% |
| | Total amount 100.0% |

Except using 2-ethylhexyl acrylate·vinyl acetate hydroxyethyl acrylate copolymer instead of 2-ethylhexyl acrylate·vinyl acetate·acrylic acid copolymer, the adhesive patch of the invention was prepared in the same way as the example 3.

### (Comparative Example 5)

| | |
|---|---|
| 2-Ethylhexyl acrylate·vinyl acetate·acrylic acid copolymer | 56.5% |
| Isopropyl myristate | 15.0% |
| Sodium acetate | 8.5% |
| Bisoprololhemifumarate | 20.0% |
| | Total amount 100.0% |

Bisoprolol hemifimarate, sodium acetate and isopropyl myristate were put in a mortar and mixed thoroughly. The mixture was mixed with a solution in which 2-ethylhexyl acrylate·vinyl acetate·acrylic acid copolymer was dissolved in ethyl acetate. After the coating solution obtained was coated on a removable film made by polyethylene terephthalate, solvent was removed by drying, followed by affixing to a backing made by polyethylene terephthalate to give the adhesive patch of the invention.

### (Example 6)

| | |
|---|---|
| 2-Ethylhexyl acrylate·vinyl acetate·acrylic acid copolymer | 18.5% |
| Polyisobutylene | 8.0% |
| Alicyclic saturated hydrocarbon resin | 39.0% |
| Liquid paraffin | 10.0% |
| Isopropyl myristate | 10.5% |
| Sodium acetate | 4.5% |
| Bisoprololhemifumarate | 10.0% |
| | Total amount 100.0% |

Bisoprolol hemifimarate, sodium acetate, liquid paraffin and isopropyl myristate were put in amortar and mixed thoroughly. The mixture was mixed with a solution in which 2-ethylhexyl acrylate·vinylacetate·acrylic acid copolymer, polyisobutylene and alicyclic saturated hydrocarbon resin (Arcon P 100, manufactured by Arakawa Chemical Industries, Ltd.) were dissolved in toluene and ethyl acetate. After the coating solution obtained was coated on a removable film made by polyethylene terephthalate, solvent was removed by drying, followed by affixing to a backing made by polyethylene terephthalate to give the adhesive patch of the invention.

### (Example 7)

| | |
|---|---|
| Duro-Tak87-2194 (manufactured by National Starch and Chemicals Co., Ltd.) | 15.0% |
| Styrene-isoprene-styrene block copolymer | 15.0% |
| Alicyclic saturated hydrocarbon resin | 39.0% |
| Liquid paraffin | 8.5% |
| Isostearyl alcohol | 8.0% |
| Sodium acetate | 4.5% |
| Bisoprolol hemifumarate | 10.0% |
| | Total amount 100.0% |

Styrene-isoprene-styrene block copolymer and alicyclic saturated hydrocarbon resin (Arcon P 100, manufactured by Arakawa Chemical Industries, Ltd.) were dissolved in toluene and mixed with Duro-Tak87-2194 (manufactured by National Starch and Chemicals Co., Ltd.). Then, to the mixture were added bisoprolol hemifimarate, isostearyl alcohol and liquid paraffin, which were put in a mortar and mixed thoroughly, and stirred to give a homogeneous solution. After the coating solution obtained was coated on a removable film made by polyethylene terephthalate, solvent was removed by drying, followed by affixing to a backing made by polyethylene terephthalate to give the adhesive patch of the invention.

### (Example 8)

| | |
|---|---|
| Duro-Tak87-2852 (manufactured by National Starch and Chemicals Co., Ltd.) | 15.0% |
| Styrene-isoprene-styrene block copolymer | 15.0% |
| Alicyclic saturated hydrocarbon resin | 40.0% |
| Liquid paraffin | 10.3% |
| Lauric diethanolamide | 5.0% |
| Lactic acid | 4.7% |
| Bisoprololhemifumarate | 10.0% |
| | Total amount 100.0% |

According to the above formula, in which as an adhesive polymer Duro-Tak87-2852 was used and as percutaneous absorption promoters lauric diethanolamide and lactic acid were added, the adhesive patch of the invention was prepared in the same way as the example 1.

### (Comparative example 1)

| | |
|---|---|
| Styrene-isoprene-styrene block copolymer | 14.5% |
| Polyisobutylene | 7.5% |
| Alicyclic saturated hydrocarbon resin | 37.5% |
| Liquid paraffin | 21.0% |
| Isopropyl myristate | 5.0% |
| Sodium acetate | 4.5% |
| Bisoprololhemifumarate | 10.0% |
| | Total amount 100.0% |

Bisoprolol hemifimarate, sodium acetate, liquid paraffin and isopropyl myristate were put in a mortar and mixed thoroughly. The mixture was mixed with a solution in which styrene-isoprene-styrene block copolymer, polyisobutylene and alicyclic saturated hydrocarbon resin (Arcon P100, manufactured by Arakawa Chemical Industries, Ltd.) were dissolved in toluene. After the coating solution obtained was coated on a removable film made by polyethylene terephthalate, solvent was removed by drying, followed by affixing to a backing made by polyethylene terephthalate to give the adhesive patch of the invention.

### (Comparative example 2)

| | |
|---|---|
| 2-Ethylhexyl acrylate·vinyl acetate copolymer | 18.5% |
| Styrene-isoprene-styrene block copolymer | 8.0% |
| Alicyclic saturated hydrocarbon resin | 39.0% |
| Liquid paraffin | 10.0% |
| Isopropyl myristate | 10.0% |
| Sodium acetate | 4.5% |
| Bisoprololhemifumarate | 10.0% |
| | Total amount 100.0% |

Except using 2-ethylhexyl acrylate·vinyl acetate copolymer instead of 2-ethylhexyl acrylate·vinyl acetate·acrylic acid copolymer, the adhesive patch of the invention was prepared in the same way as the example 3.

### Test 1: Skin penetration test in hairless mice

A back part skin of a hairless mouse was removed, and the dermal side was placed to a receptor layer side and installed in a flow-through cell (5 cm²), in which warm water set up so that the temperature of the skin surface became 32°C was circulated around the outer part. Each of the adhesive patches obtained in the examples 1-8 as well as the comparative examples 1-2 was pasted on the stratum corneum side, and samplings were carried out at every 40 minutes for 8 hours at a rate of 5 ml/hour using pH 7.4 phosphate buffer saline in the receptor phase. As to the receptor solutions obtained at each time, the flow amounts were accurately measured, and the drug concentrations were measured by a high-performance liquid chromatography. The penetration rate per hour was calculated by measured values of the flow amount and the drug concentration, and the maximum skin penetration rate in each example was determined. The results are shown in Table 1.

### Test 2: Adhesive force test

As for the patches obtained, an adhesive force was measured by a probetack tester and a peel measuring instrument, and a cohesive force by a creep measuring instrument respectively, and based on the following criteria:
A: Sufficient in both the adhesive force and the cohesive force,
B: Although at least one of the adhesive force and the cohesive force is insufficient, it is usable by use of a sheet-type cover,
C: Both the adhesive force and the cohesive force are insufficient,
   the evaluation was carried out. The results are shown in Table 1.

### Test 3: Drug-content stability test

As for the patches obtained, a stability test with the passage of time for a drug content per one sheet of the preparation was carried out. A storage condition was at 40°C for 3 months, the test was carried out in a formof sealing it in an aluminum package. The drug content of bisoprolol was measured by a HPLC method. The results are shown in Table 1.

**[Table 1]**

| | Skin penetration rate | Adhesive properties | Crystal deposition | Drug-content stability (40°C-3 months) Against initial value [%] |
|---|---|---|---|---|
| Example 1 | 4.3 µg/cm²/hr | A | No | 100.2% |
| Example 2 | 4.0 µg/cm²/hr | A | No | 99.0% |
| Example 3 | 54.3 µg/cm²/hr | A | No | 98.6% |
| Example 4 | 76.8 µg/cm²/hr | A | No | 78.4% |
| Example 5 | 52.4 µg/cm²/hr | B | No | 98.3% |
| Example 6 | 39.6 µg/cm²/hr | A | No | 98.5% |
| Example 7 | 34.0 µg/cm²/hr | A | No | 99.1% |
| Example 8 | 45.6 µg/cm²/hr | A | No | 98.6% |
| Comparative example 1 | 24.5 µg/cm²/hr | B | Yes | 100.3% |
| Comparative example 2 | 67.7 µg/cm²/hr | B | No | 88.9% |

### Calculation method for human blood plasma concentration profile

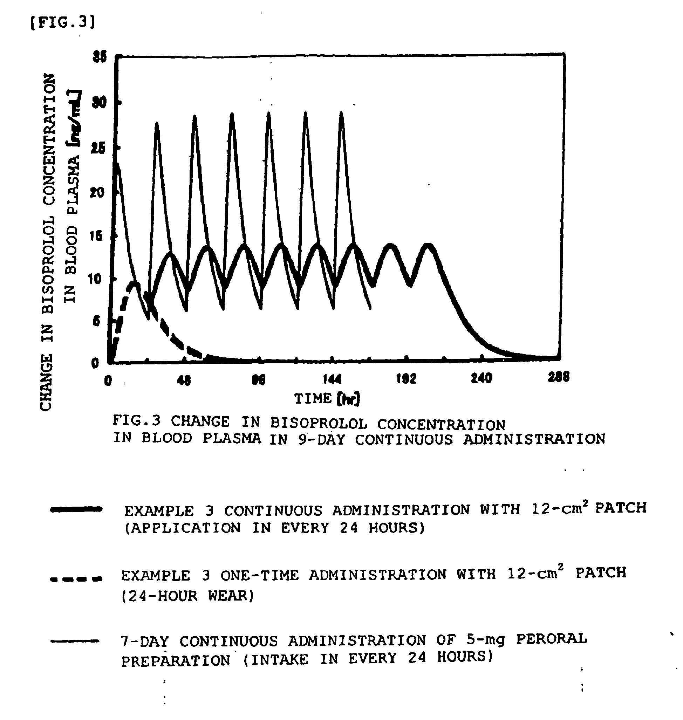

Apharmacokinetic parameter in human peroral administration of bisoprolol hemifumarate was determined by WinNonlin (Scientific Consulting Inc.), a pharmacokinetic analysis soft, using known data of peroral preparations (5mg). Human blood plasma concentrations at the time of single administration and at the time of continuous administration were calculated by SKIN-CADTM Professional Edition ver.2.0 ( Ehive-Communication Co., Ltd.), a percutaneous absorption estimation system, using the test results of the human skin penetration (Fig. 2) obtained by those parameters and that obtained in the example 3. The results are shown in Fig.3. Further, a preparation area was made 12cm². In addition, for comparison, the concentration in blood plasma for 5-mg peroral preparation was shown together.

As is evident from the graphs in Fig.3, in a novel bisoprolol-containing adhesive patch of the invention , a drug penetration rate through the skin is high, and additionally, a drug concentration in blood does not transiently rise, almost keeping constant, whereby it can be administered to human body safely and effectively for a long period compared with a peroral preparation.

According to the adhesive patch of the invention, bisoprolol can efficiently be absorbed into circulating blood through the skin. In addition, a side effect of the gastrointestinal system observed in case of a peroral administration and a side effect which can occur with a rapid increase of the blood concentration can also be avoided, therefore, it is very effective as an external preparation aiming at the percutaneous application.

### [Brief Description of Drawing]

Figure 1 shows a descriptive drawing of a structure of an adhesive patch of the invention.
Figure 2 shows a drawing indicating a human skin penetration rate when pasting an adhesive patch of the invention.

Figure 2 shows a drawing of a change in bisoprolol concentration in blood plasma when continuously administering an adhesive patch of the invention.

## Claims

1. An adhesive patch having a pressure-sensitive adhesive layer comprising bisoprolol and/or a pharmaceutically acceptable salt thereof, wherein the composition of pressure-sensitive adhesive layer contains an acrylic polymer obtained by copolymerizing a (meth)acrylic ester with a (meth)acrylic acid comprising a carboxyl group and an elastomeric polymer.

2. The adhesive patch according to claim 1, wherein the acrylic polymer contains substantially no alcoholic hydroxyl group in molecules.

3. The adhesive patch according to claims 1 or 2, wherein the (meth)acrylic ester constituting the acrylic polymer is 2-ethylhexyl acrylate.

4. The adhesive patch according to any one of claims 1 to 3, wherein the acrylic polymer is a 2-ethylhexyl acrylate·vinyl acetate·acrylic acid copolymer.

5. The adhesive patch according to any one of claim 1 to 4, wherein the elastomeric polymer is at least one kind selected from a group consisting of styrene-isoprene-styrene block copolymer, polyisobutylene, isoprene rubber, styrene-butadine-styrene block copolymer, styrene-butadine rubber and silicone rubber.

6. The adhesive patch according to any one of claims 1 to 5, wherein the percutaneous absorption promoter is at least one kind selected from a group consisting of lauryl alcohol, myristyl alcohol, oleyl alcohol, isostearyl alcohol, diethyl sebacate, lauric acid diethanolamide, isopropyl myristate, glycerol monocaprate, glycerol monolaurate, glycerol monooleate, sorbitan monolaurate, propylene glycol monolaurate, polyoxyethylene lauryl ether, pyrothiodecane, an organic acid and a pharmaceutically acceptable salt thereof.

7. The adhesive patch according to claim 6, wherein the organic acid and/or the pharmaceutically acceptable salt thereof are at least one kind selected from a group consisting of acetic acid, propionic acid, lactic acid, salicylic acid and pharmaceutically acceptable salts thereof.

8. The adhesive patch according to claim 6, wherein the percutaneous absorption promoter is isopropyl myristate.

9. The adhesive patch according to claim 8, wherein it contains further sodium acetate as the percutaneous absorption promoter.

10. The adhesive patch according to any one of claims 1 to 9, wherein the drug is bisoprolol hemifumarate.

11. The adhesive patch according to any one of claims 1 to 10, wherein the penetration rate of bisoprolol through the skin is 3-300 µg/h·cm².

## Patentansprüche

1. Ein Klebepflaster mit einer Haftkleberschicht, das Bisoprolol und/oder ein pharmazeutisch akzeptables Salz davon beinhaltet, wobei die Zusammensetzung der Haftkleberschicht ein Acrylpolymer, das durch Copolymerisieren eines (Meth)acrylesters mit einer eine Carboxylgruppe beinhaltenden (Meth)acrylsäure erhalten wurde, und ein elastomeres Polymer enthält.

2. Klebepflaster gemäß Anspruch 1, wobei das Acrylpolymer im Wesentlichen keine alkoholische Hydroxylgruppe in Molekülen enthält.

3. Klebepflaster gemäß den Ansprüchen 1 oder 2, wobei der das Acrylpolymer ausmachende (Meth)acrylester 2-Ethylhexylacrylat ist.

4. Klebepflaster gemäß einem der Ansprüche 1 bis 3, wobei das Acrylpolymer ein 2-Ethylhexylacrylat-Vinylacetat-Acrylsäure-Copolymer ist.

5. Klebepflaster gemäß einem der Ansprüche 1 bis 4, wobei das elastomere Polymer mindestens eine Art ist, die aus einer Gruppe ausgewählt ist, die aus Styren-Isopren-Styren-Blockcopolymer, Polyisobutylen, Isoprenkautschuk, Styren-Butadien-Styren-Blockcopolymer, Styren-Butadienkautschuk und Silikonkautschuk besteht.

6. Klebepflaster gemäß einem der Ansprüche 1 bis 5, wobei der Förderer der perkutanen Absorption mindestens eine Art ist, die aus einer Gruppe ausgewählt ist, die aus Laurylalkohol, Myristylalkohol, Oleylalkohol, Isostearylalkohol, Diethylsebacat, Laurinsäurediethanolamid, Isopropylmyristat, Glycerolmonocaprat, Glycerolmonolaurat, Glycerolmonooleat, Sorbitanmonolaurat, Propylenglykolmonolaurat, Polyoxyethylenlaurylether, Pyrothiodecan, einer organischen Säure und einem pharmazeutisch akzeptablen Salz davon besteht.

7. Klebepflaster gemäß Anspruch 6, wobei die organische Säure und/oder das pharmazeutisch akzeptable Salz davon mindestens eine Art ist/sind, die aus einer Gruppe ausgewählt ist, die aus Essigsäure, Propionsäure, Milchsäure, Salicylsäure und pharmazeutisch akzeptablen Salzen davon besteht.

8. Klebepflaster gemäß Anspruch 6, wobei der Förderer der perkutanen Absorption Isopropylmyristat ist.

9. Klebepflaster gemäß Anspruch 8, wobei es ferner Natriumacetat als den Förderer der perkutanen Absorption beinhaltet.

10. Klebepflaster gemäß einem der Ansprüche 1 bis 9, wobei das Arzneimittel Bisoprololhemifumarat ist.

11. Klebepflaster gemäß einem der Ansprüche 1 bis 10, wobei die Durchdringungsrate des Bisoprolols durch die Haut 3-300 µg/h·cm² beträgt.

## Revendications

1. Un patch adhésif présentant une couche adhésive sensible à la pression comprenant du bisoprolol et/ou un sel acceptable d'un point de vue pharmaceutique de celui-ci, dans lequel la composition de couche adhésive sensible à la pression contient un polymère acrylique obtenu par copolymérisation d'un ester (méth)acrylique avec un acide (méth)acrylique comprenant un groupe carboxyle et un polymère élastomère.

2. Le patch adhésif selon la revendication 1, dans lequel le polymère acrylique ne contient substantiellement aucun groupe hydroxyle alcoolique dans les molécules.

3. Le patch adhésif selon les revendications 1 ou 2, dans lequel l'ester (méth)acrylique constituant le polymère acrylique est l'acrylate de 2-éthylhexyle.

4. Le patch adhésif selon l'une quelconque des revendications 1 à 3, dans lequel le polymère acrylique est un copolymère d'acide acrylique acétate de vinyle acrylate de 2-éthylhexyle.

5. Le patch adhésif selon l'une quelconque des revendications 1 à 4, dans lequel le polymère élastomère est au moins une espèce sélectionnée dans un groupe consistant en copolymère bloc styrène-isoprène-styrène, polyisobutylène, caoutchouc isoprène, copolymère bloc styrène-butadiène-styrène, caoutchouc styrène-butadiène et caoutchouc de silicone.

6. Le patch adhésif selon l'une quelconque des revendications 1 à 5, dans lequel le promoteur d'absorption percutanée est au moins une espèce sélectionnée dans un groupe consistant en alcool laurylique, alcool myristylique, alcool oléylique, alcool isostéarylique, sébacate de diéthyle, diéthanolamide d'acide laurylique, myristate d'isopropyle, monocaprate de glycérol, monolaurate de glycérol, monooléate de glycérol, monolaurate de sorbitan, monolaurate de propylèneglycol, éther laurylique de polyoxyéthylène, pyrothiodécane, un acide organique et un sel acceptable d'un point de vue pharmaceutique de celui-ci.

7. Le patch adhésif selon la revendication 6, dans lequel l'acide organique et/ou le sel acceptable d'un point de vue pharmaceutique de celui-ci sont au moins une espèce sélectionnée dans un groupe consistant en acide acétique, acide propionique, acide lactique, acide salicylique et des sels acceptables d'un point de vue pharmaceutique de ceux-ci.

8. Le patch adhésif selon la revendication 6, dans lequel le promoteur d'absorption percutanée est le myristate d'isopropyle.

9. Le patch adhésif selon la revendication 8, lequel contient en outre de l'acétate de sodium en tant que promoteur d'absorption percutanée.

10. Le patch adhésif selon l'une quelconque des revendications 1 à 9, dans lequel le médicament est l'hémifumarate de bisoprolol.

11. Le patch adhésif selon l'une quelconque des revendications 1 à 10, dans lequel la vitesse de pénétration du bisoprolol dans la peau est de 3 à 300 µg/h·cm².
